(19) Europäisches Patentamt
European Patent Office
Office européen des brevets

(11) **EP 1 098 593 B1**

(12) **EUROPÄISCHE PATENTSCHRIFT**

(45) Veröffentlichungstag und Bekanntmachung des Hinweises auf die Patenterteilung:
**12.05.2004 Patentblatt 2004/20**

(21) Anmeldenummer: **99940036.9**

(22) Anmeldetag: **22.07.1999**

(51) Int Cl.7: **A61B 5/11**, A61B 5/048

(86) Internationale Anmeldenummer:
**PCT/EP1999/005250**

(87) Internationale Veröffentlichungsnummer:
**WO 2000/004827 (03.02.2000 Gazette 2000/05)**

(54) **VORRICHTUNG ZUR BESTIMMUNG DER SCHLAFTIEFE**

DEVICE FOR DETERMINING DEPTH OF SLEEP

DISPOSITIF PERMETTANT DE DETERMINER LA PROFONDEUR DU SOMMEIL

(84) Benannte Vertragsstaaten:
**AT BE CH CY DE DK ES FI FR GB GR IE IT LI LU MC NL PT SE**

(30) Priorität: **24.07.1998 DE 19833497**

(43) Veröffentlichungstag der Anmeldung:
**16.05.2001 Patentblatt 2001/20**

(73) Patentinhaber: **Dimpfel, Wilfried, Prof. Dr.
35415 Pohlheim (DE)**

(72) Erfinder: **Dimpfel, Wilfried, Prof. Dr.
35415 Pohlheim (DE)**

(74) Vertreter: **HOFFMANN - EITLE
Patent- und Rechtsanwälte
Arabellastrasse 4
81925 München (DE)**

(56) Entgegenhaltungen:
**WO-A-93/07804          US-A- 5 699 808**

EP 1 098 593 B1

**Beschreibung**

Die vorliegende Erfindung betrifft eine Vorrichtung zur Bestimmung der Schlaftiefe, insbesondere während der Narkose bei einem therapeutischen/chirurgischen Eingriff.

Die Bestimmung der Schlaftiefe sowohl bei physiologischem als auch bei medikamenten-induziertem Schlaf erfolgt in der Regel visuell durch erfahrene Experten, die das EEG eines Patienten auswerten und einzelnen 20 bis 30 Sekunden langen Zeitabschnitten Werte zuweisen, die die Schlaftiefe wiedergeben. Die Bewertung basiert auf Arbeiten von Rechtschaffen & Kales aus dem Jahre 1969 und ist heutzutage der einzige allgemein anerkannte Standard für die Beurteilung und Ermittlung der Schlaftiefe des Menschen auf der Grundlage eines EEG.

Es ist offensichtlich, daß sich dieses Verfahren nicht dafür eignet, in größerem Maßstab oder während eines therapeutischen/chirurgischen Eingriffs durchgeführt zu werden, für den der Patient in einen medikamenten-induzierten Schlaf (Narkose) versetzt wird. Aus diesem Grund sind zahlreiche Versuche unternommen worden, die Schlaftiefe auf der Basis des EEG-Signals zu bestimmen. Insbesondere wurden dazu EEG-Signale transformiert (Fast-Fourrier-Transformation) und die Darstellung des EEG-Signals im Frequenzbereich einer Auswertung unterzogen. Besondere Berücksichtigung fanden dabei Frequenzen, die allgemein als $\Delta$-Wellen bezeichnet wurden, und die in einem Bereich von etwa 0 bis 3,5 Hz lagen. Diese sehr langwelligen Anteile des EEG-Signals wurden mit dem Bewußtseinszustand "Schlaf" in Verbindung gebracht und Methoden entwickelt, die auf der Grundlage dieser Frequenzen eine Bestimmung der Schlaftiefe ermöglichen sollten. Die Ergebnisse wurden dadurch nachgeprüft, daß die automatisch bestimmte Schlaftiefe verglichen wurde mit der von Experten visuell bestimmten Schlaftiefe nach dem anerkannten Verfahren von Rechtschaffen & Kales. Die bisher eingesetzten Methoden führten aber zu keinen verläßlichen oder nur sehr eingeschränkt verwendbaren Ergebnissen, so daß letztlich eine zuverlässige vollautomatische Bestimmung der Schlaftiefe bis heute nicht möglich ist.

Neben den zuvor beschriebenen Bemühungen, den Bewußtseinszustand "Schlaf" allgemeingültig auf automatisierbare Weise zu bestimmen, gab es Versuche, insbesondere medikamenten-induzierten Schlaf zu bewerten. Dabei wurden teilweise einzelne Frequenzbereiche auch außerhalb des Bereichs der $\Delta$-Wellen untersucht. Auch dabei war der Erfolg nur mäßig und überdies auf das jeweils untersuchte Medikament beschränkt, so daß die Ergebnisse nicht auf andere Medikamente übertragen werden konnten. Auch die Validierung der Ergebnisse über eine visuelle Bewertung auf der Basis von Rechtschaffen & Kales zeigte Defizite, so daß von einer zuverlässigen Bewertung des menschlichen Schlafes kaum gesprochen werden kann.

Nicht nur für wissenschaftliche oder medizinische Untersuchungen und Anwendungen ist die Kenntnis der Schlaftiefe von Interesse. Das Ausbleiben des medikamenteninduzierten Schlafes während eines therapeutischen/chirurgischen Eingriffes stellt eine für den Nicht-betroffenen kaum vorstellbare Belastung des Patienten dar. Dies gilt umso mehr, wenn neben dem Ausbleiben des medikamenten-induzierten Schlafes auch die medikamenteninduzierte Schmerzunempfindlichkeit nicht erzielt wird und gleichzeitig, die bei Narkosen regelmäßig vorgesehene Paralysierung der Muskelaktivitäten erfolgreich induziert wird. Für die Patienten bedeutet dies letztlich, daß ein therapeutischer/chirurgischer Eingriff vorgenommen wird, ohne daß der Patient schläft und schmerzunempfindlich ist, aber auch ohne daß er in der Lage ist, auf seinen Zustand aufmerksam zu machen. Durch eine zuverlässige Erkennung des Schlafes, die vollautomatisch durchgeführt wird und während eines therapeutischen/chirurgischen Eingriffs und der damit verbundenen Narkose mitläuft, kann hier Abhilfe geschaffen werden.

Aber unabhängig von den zuvor geschilderten und üblicherweise seltenen Fall des Narkoseversagens besteht grundsätzliches Interesse an einem zuverlässigen Verfahren zur qualifizierten und quantitativen Bestimmung des Bewußtseinszustands Schlaf, so daß die der Erfindung zugrunde liegende Aufgabe darin zu sehen ist, eine Vorrichtung zu schaffen, mit der die Schlaftiefe zuverlässig und vollautomatisch auf der Basis des EEG-Signals bestimmt werden kann.

Aus US-A-5 699 808 ist eine Vorrichtung zur Bestimmung der Schlaftiefe bekannt mit einer Transformationseinrichtung, der ein Ausgangssignal einer EEG-Messapparatur zugeführt wird und die das zugeführte EEG-Signal in einen Frequenzbereich transformiert, der die Frequenzbereiche a, b und c umfaßt und einer Verarbeitungseinrichtung, der das in den Frequenzbereich transformierte Signal zugeführt wird und die für mindestens zwei Frequenzbereiche repräsentative Werte ermittelt und daraus einen Schlaftiefe-Index ermittelt. Als Frequenzbereiche werden Bereiche a von 3,5 bis 7,5 Hz, b von 7,7 bis 12, 5 Hz und c von 12.5-25 Hz genannt.

Gelöst wird diese Aufgabe durch eine Vorrichtung mit den Merkmalen des Patentanspruchs 1. Vorteilhafte Ausgestaltungen ergeben sich aus den Unteransprüchen.

Die Erfindung basiert auf der Erkenntnis, daß von drei charakteristischen Bereichen des im Frequenzbereich dargestellten EEG-Signals zumindest zwei herangezogen werden müssen, um einen Schlaftiefe-Index $S_F$ zu bestimmen. Entscheidend ist, daß das EEG-Signal in Bezug auf diese Bereiche untersucht wird und unter Berücksichtigung von zumindest zwei, vorzugsweise aber aller drei Bereiche der Schlaftiefe-Index $S_F$ ermittelt wird.

Im folgenden wird ein Ausführungsbeispiel der erfindungsgemäßen Vorrichtung unter Bezugnahme auf die Figuren genauer beschrieben, in denen zeigt:

Fig. 1     schematisch den Aufbau eines Ausführungsbeispiels der erfindungsgemäßen Vorrichtung;

Fig. 2     schematisch den Verlauf des EEG-Signals im Frequenzbereich sowie die erfindungsgemäß zu berücksichtigenden Bereiche.

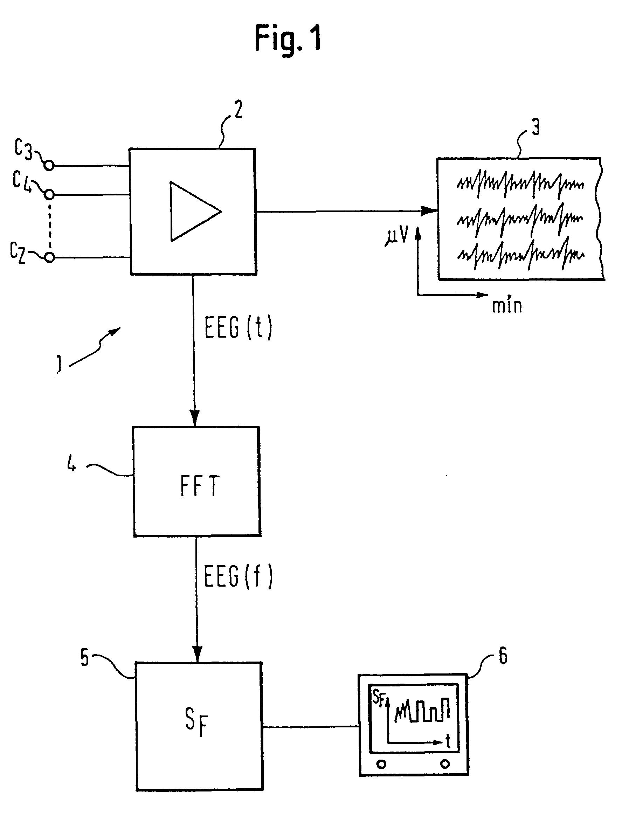

In Fig. 1 ist schematisch ein Ausführungsbeispiel der erfindungsgemäßen Vorrichtung dargestellt. Ein Ausführungsbeispiel umfaßt eine EEG-Meßapparatur 1, von der in Fig. 1 nur die Elektroden C3 und C4 sowie die Elektrode $C_Z$ dargestellt sind. Üblicherweise umfaßt eine Standard-EEG-Meßapparatur 15-25 Elektroden zusätzlich zu der Elektrode $C_Z$. Die Ausgangssignale der Elektroden werden einer Verstärkereinrichtung 2 zugeführt, die die Elektrodensignale so verstärkt und verarbeitet, daß damit ein Schreiber (nicht dargestellt) angesteuert werden kann, der das EEG 3 schreibt. In der Verstärkereinrichtung 2 sind vorzugsweise Entstörfilter (nicht dargrstellt) vorgesehen, die in den zugeführten Elektrodensignalen die Anteile im Bereich von 0 bis 4,5 Hz und von 7 bis 12,25 Hz unterdrücken. Bei der erfindungsgemäßen Vorrichtung wird ein EEG-Signal, vorzugsweise das der Elektroden $C_3$ und/ oder $C_4$, aus der EEG-Meßapparatur 1 abgeleitet und einer Transformationseinrichtung 4 zur Durchführung einer Fourriertransformation (Fast Fourrier Transformation, FFT) zugeführt. Die Verbindung zwischen der EEG-Meßapparatur 2 und der Transformationseinrichtung 4 kann auf verschiedene Weise hergestellt werden, vorzugsweise über eine optische Verbindung, beispielsweise eine Glasfaser, so daß auch bei größeren Übertragungsstrecken eine Störung des übertragenen EEG-Signals sicher ausgeschlossen wird.

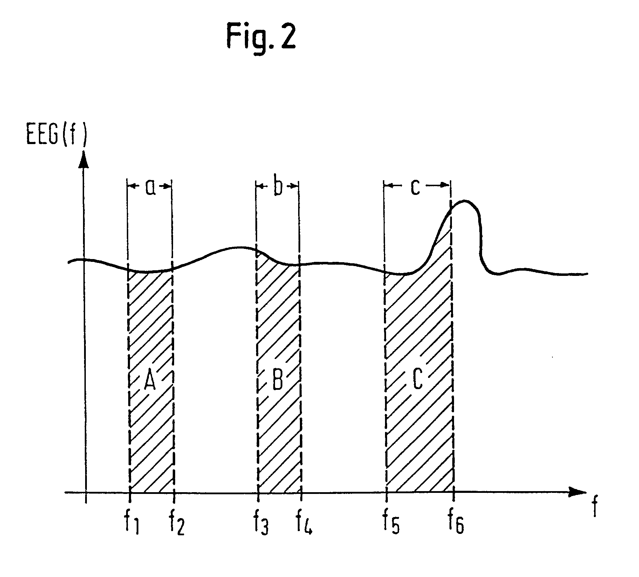

Das Ausgangssignal der Transformationseinrichtung 4 gibt das Fourrier-transformiete EEG-Signal an eine Verarbeitungseinrichtung 5 weiter, die das nun im Frequenzbereich vorliegende EEG-Signal EEG(f) weiter verarbeitet. Dazu werden drei Frequenzbereiche des transformierten EEG-Signals genauer untersucht. Die Frequenzbereiche sind in Fig. 2 dargestellt, wobei der Frequenzbereich a von 4,75 bis 6,75 Hz, der Frequenzbereich b von 12,75 bis 18,5 Hz und der Frequenzbereich c von 18,75 bis 35,0 Hz reicht. Die Verarbeitungseinrichtung 5 bestimmt für jeden der Frequenzbereiche einen repräsentativen Wert A, B und C und ermittelt daraus den die Schlaftiefe kennzeichnenden Index

$$S_F = \frac{A + B}{C}.$$

Der Schlaftiefe-Index $S_F$ reflektiert die Schlaftiefe des Patienten zuverlässig, wie in einer Validierungsstudie gegenüber den nach Rechtschaffen & Kales ermittelten Schlafzuständen nachgewiesen werden konnte.

Die für die drei Frequenzbereiche a, b und c repräsentativen Werte A, B und C können auf verschiedene Weise gewonnen werden. Entscheidend ist, daß zumindest zwei der Frequenzbereiche a, b und c, vorzugsweise aber alle drei, vollständig oder teilweise bei der Bestimmung der repräsentativen Werte berücksichtigt werden. Ein Verfahren zur Bestimmung repräsentativer Werte besteht in der Integration des transformierten EEG-Signals von der Anfangsfrequenz bis zur Endfrequenz des jeweiligen Bereichs bzw. eines Ausschnitts innerhalb des Bereichs.

In Fig. 2 geben die schraffierten Flächen die repräsentativen Werte A, B und C wieder, die der Integration des transformierten EEG Signals in den Frequenzbereichen a, b und c ermittelt werden. Als repräsentativer Wert eignet sich grundsätzlich aber auch ein Mittelwert des transformierten EEG-Signals, der über die einzelnen Frequenzbereiche a, b und c bestimmt wird. Eine andere Form der Berechnung basiert auf der Leistungsverteilung h(f) des Signals, vorzugsweise des gefilterten Signals, nach folgendem Modus:

$$S = \frac{\int h(f) \cdot f df}{\int h(f) \cdot df}$$

Ferner kann jedes mathematische Verfahren mit ähnlicher Aussage herangezogen werden.

Wie in Fig. 1 gezeigt, umfaßt das hier beschriebene Ausführungsbeispiel der erfindungsgemäßen Vorrichtung einen Monitor 6 zur Darstellung des Schlaftiefe-Index $S_F$. Neben einer Darstellung auf einem Monitor kann das Signal $S_F$ auch für andere Zwecke, beispielsweise eine Alarmierung, genutzt werden. Das Signal $S_F$ kann auch dem (nicht dargestellten) EEG-Schreiber zugeführt werden, so daß auf dem EEG 3 auch der Schlaftiefe-Index $S_F$ wiedergegeben wird.

Zu beachten ist, daß die erfindungsgemäße Vorrichtung einen Index angibt, der in dieser Form bislang nicht bestimmt wurde und der als Schlaftiefe-Index $S_F$ bezeichnet wird. Dieser Index unterscheidet sich von den bisher ermittelten Werten und gestattet die Bestimmung physiologischen und medikamenten-induzierten Schlafs, unabhängig vom eingesetzten Medikament.

Die Transformationseinrichtung und die Verarbeitungseinrichtung sind vorzugsweise in einem Personal Computer zusammengefaßt, der auch die Rechenleistung zur Verfügung stellen kann, die für die Transformation des EEG-Signals in den Frequenzbereich und/oder die Verarbeitung des transformierten EEG-Signals, d.h. die Bestimmung des Schlaftiefe-Index $S_F$ erforderlich ist.

**Patentansprüche**

1.     Vorrichtung zur Bestimmung der Schlaftiefe mit

- einer Transformationseinrichtung (4), der ein Ausgangssignal (EEG(t)) einer EEG-Messapparatur (1) zugeführt wird und die das zugeführte EEG-Signal in einen Frequenzbereich, der die Frequenzbereiche a, b und c umfasst, transformiert, und

- einer Verarbeitungseinrichtung (5), der das in den Frequenzbereich transformierte EEG-Signal (EEG(f)) von der Transformationseinrichtung (4) zugeführt wird und die für den Frequenzbereich a von 4,75 bis 6,75 Hz, den Frequenzbereich b von 12,75 bis 18,5 Hz und den Frequenzbereich c von 18,75 bis 35,0 Hz oder zumindest zwei der Frequenzbereiche repräsentative Werte A, B und C ermittelt und daraus einen Schlaftiefe-Index $S_F$ bestimmt.

2. Vorrichtung nach Anspruch 1,
**dadurch gekennzeichnet, daß**
die Verarbeitungseinrichtung (5) den Schlaftiefe-Index $S_F$ bestimmt als Quotienten aus der Summe der repräsentativen Werte A und B und dem repräsentativen Wert C

$$(S_F = \frac{A + B}{C}).$$

3. Vorrichtung nach Anspruch 1 oder 2,
**dadurch gekennzeichnet, daß**
die Verarbeitungseinrichtung (5) die repräsentativen Werte A, B und C durch Integration des EEG-Signals im Frequenzbereich ermittelt.

4. Vorrichtung nach einem der Ansprüche 1 oder 2,
**dadurch gekennzeichnet, daß**
die Verarbeitungseinrichtung (5) die repräsentativen Werte A, B und C durch Mittlung des EEG-Signals im Frequenzbereich ermittelt.

5. Vorrichtung nach einem der Ansprüche 1 oder 2,
**dadurch gekennzeichnet, daß**
die Verarbeitungseinrichtung (5) die repräsentativen Werte A, B und C auf der Basis der Leistungsverteilung h(f) des Signals nach dem Modus

$$S = \frac{\int h(f)\cdot f df}{\int h(f)\cdot df}$$

ermittelt.

6. Vorrichtung nach einem der vorangegangenen Ansprüche,
**dadurch gekennzeichnet, daß**
die Transformationseinrichtung (4) das zugeführte EEG-Signal (EEG(t)) mittels einer schnellen FourierTransformation (FFT) in den Frequenzbereich transformiert.

7. Vorrichtung nach einem der vorangegangenen Ansprüche,
**gekennzeichnet durch**
zumindest eine Entstöreinrichtung, die bei den Elektrodensignalen der EEG-Meßapparatur (1) die Anteile im Bereich von 0 bis 4,5 Hz und 7 bis 12,25 Hz unterdrückt.

**Claims**

1. Device for determining the depth of sleep having

- a transformation device (4), to which is supplied an output signal (EEG(t)) of an EEG measuring apparatus (1) and which transforms the EEG signal supplied into a frequency range which comprises the frequency ranges a, b and c, and
- a processing device (5), to which is supplied the EEG signal (EEG (f)) from the transformation device (4) transformed into the frequency range and which ascertains representative values A, B and C for the frequency range a from 4.75 to 6.75 Hz, the frequency range b from 12.75 to 18.5 Hz and the frequency range c from 18.75 to 35.0 Hz or at least two of the frequency ranges and determines therefrom a depth of sleep index $S_F$.

2. Device according to claim 1, **characterised in that** the processing device (5) determines the depth of sleep index $S_F$ as a quotient of the sum of the representative values A and B and the representative value C

$$(S_F = \frac{A + B}{C}).$$

3. Device according to claim 1 or 2, **characterised in that** the processing device (5) ascertains the representative values A, B and C by integration of the EEG signal in the frequency range.

4. Device according to one of claims 1 or 2, **characterised in that** the processing device (5) ascertains the representative values A, B and C by averaging the EEG signal in the frequency range.

5. Device according to one of claims 1 or 2, **characterised in that** the processing device (5) ascertains the representative values A, B and C on the basis of the power distribution h(f) of the signal according to the mode

$$S = \frac{\int (f)\cdot f df}{\int h(f)\cdot df}.$$

6. Device according to one of the preceding claims, **characterised in that** the transformation device (4) transforms the EEG signal (EEG (t)) supplied by means of a rapid Fourier transformation (FFT) into the frequency range.

7. Device according to one of the preceding claims, **characterised by** at least one anti-interference device, which suppresses the proportions in the range from 0 to 4.5 Hz and 7 to 12.25 Hz for the electrode signals of the EEG measuring apparatus (1).

**Revendications**

1. Dispositif permettant de déterminer la profondeur du sommeil, comprenant :

   - un dispositif de transformation (4), auquel est amené un signal de sortie (EEG(t)) venant d'un appareil de mesure EEG (1) et qui transforme le signal EEG amené, dans une plage de fréquences comprenant les plages de fréquence a, b et c, et
   - un dispositif de traitement (5), auquel le signal EEG (EEG(f)), ayant été transformé dans la plage de fréquences, venant du dispositif de transformation (4), est amené, et qui détermine, pour la plage de fréquences a, allant de 4,75 à 6,75 Hz, la plage de fréquences b, allant de 12,75 à 18,5 Hz, et la plage de fréquences c, allant de 18,75 à 35,0 Hz, ou pour au moins deux des plages de fréquence, des valeurs représentatives A, B et C, et, à partir de là, détermine un index de profondeur de sommeil $S_F$.

2. Dispositif selon la revendication 1, **caractérisé en ce que** le dispositif de traitement (5) détermine l'indice de profondeur de sommeil $S_F$ sous forme de quotients à partir de la somme des valeurs A et B représentatives et de la valeur C représentative.

$$(S_F \frac{A + B}{C}).$$

3. Dispositif selon la revendication 1 ou 2, **caractérisé en ce que** le dispositif de traitement (5) détermine les valeurs A, B, et C représentatives par une intégration du signal EEG dans la plage des fréquences.

4. Dispositif selon la revendication 1 ou 2, **caractérisé en ce que** le dispositif de traitement (5) détermine les valeurs A, B, et C représentatives, par formation de la moyenne du signal EEG dans la plage des fréquences.

5. Dispositif selon la revendication 1 ou 2, **caractérisé en ce que** le dispositif de traitement (5) détermine les valeurs A, B, et C représentatives, sur la base de la distribution de puissance h(f) du signal, selon le mode

$$S = \frac{\int h(f).fdf}{\int h(f).df}$$

6. Dispositif selon l'une des revendications précédentes, **caractérisé en ce que** le dispositif de transformation (4) transforme le signal EEG (EEG(t)) amené, au moyen d'une transformation de Fourier rapide (FFT) dans la plage des fréquences.

7. Dispositif selon l'une des revendications précédentes, **caractérisé par** au moins un dispositif de déparasitage, qui supprime les composantes dans la plage de 0 à 4,5 Hz et de 7 à 12,25 Hz, pour les signaux d'électrodes de l'appareil de mesure EEG (1).

# Fig. 1

# Fig. 2